# EUROPEAN PATENT APPLICATION

(11) **EP 4 736 748 A1**
(43) Date of publication of application: **06.05.2026**
(21) Application number: 25210950.9
(22) Date of filing: 24.10.2025
(51) Int. Cl.: A61B 5/00, A61N 1/05, A61B 5/12, A61N 1/36

(54) **STATE-BASED INITIATION OF EVOKED RESPONSE-BASED MEASUREMENT TESTS DURING AN ELECTRODE LEAD INSERTION PROCEDURE**

(30) Priority: 30.10.2024 US 202418932021
(71) Applicant: Advanced Bionics, LLC, Valencia CA 91355 (US)
(72) Inventor: Koka, Kanthaiah, Valencia (US); Spahr, Anthony J., Newhall (US); Ashiri, Mehrangiz, Winnipeg (CA)
(74) Representative: Schwan Schorer & Partner mbB

(57) **Abstract**

An illustrative diagnostic system may perform a process comprising: performing, during a lead insertion procedure during which an electrode lead is inserted into a cochlea of a recipient of a cochlear implant, a first evoked response-based measurement test that generates first result data; determining that the first result data satisfies a first condition; performing, based on the first result data satisfying the first condition and during the lead insertion procedure, a second evoked response-based measurement test that generates second result data, the second evoked response-based measurement test being of a different type than the first evoked response-based measurement test; and performing, based on the second result data, an operation associated with the lead insertion procedure.

## Description

### BACKGROUND INFORMATION

During an insertion procedure in which an electrode lead is placed within the cochlea, it may be desirable to ascertain an insertion state of the electrode lead. For example, it may be desirable to determine and convey in real-time to a surgeon performing the insertion procedure when an electrode on the electrode lead passes a particular characteristic frequency location within the cochlea, when an electrode on the electrode lead is within a vicinity of a cluster of hair cells, and/or when the electrode lead is possibly causing trauma to a structure of the cochlea.

### BRIEF DESCRIPTION OF THE DRAWINGS

The accompanying drawings illustrate various embodiments and are a part of the specification. The illustrated embodiments are merely examples and do not limit the scope of the disclosure. Throughout the drawings, identical or similar reference numbers designate identical or similar elements.
Fig. 1 shows an illustrative cochlear implant system according to principles described herein.
Fig. 2 shows an illustrative configuration of the cochlear implant system of Fig. 1.
Fig. 3 shows an illustrative diagnostic system according to principles described herein.
Figs. 4-5 show illustrative implementations of the diagnostic system of Fig. 3 according to principles described herein.
Figs. 6A-6F show illustrative insertion procedure in which an electrode lead is inserted into a cochlea of a recipient according to principles described herein.
Fig. 7 illustrates an illustrative method that may be used to perform state-based initiation of evoked response-based measurement tests during an electrode lead insertion procedure.
Fig. 8 shows an implementation of the diagnostic system of Fig. 3.
Fig. 9 illustrates an illustrative method.
Figs. 10-14 shows an illustrative multi tone-based insertion depth monitoring procedure.
Fig. 15 illustrates an exemplary computing device according to principles described herein.

### DETAILED DESCRIPTION

Described herein is a state-based heuristic in which different types of evoked response-based measurement tests are used during an electrode lead insertion procedure to assist in performing the electrode lead insertion procedure. For example, a diagnostic system may initially perform a first evoked response-based measurement test during the electrode lead insertion procedure. Based on the results of the first evoked response-based measurement test, the diagnostic system may automatically initiate a different type of evoked response-based measurement test in place of the first evoked response-based measurement test. This second evoked response-based test may be selected to confirm the results of the first test and/or supplement the results of the first test in any other manner. The results data from the second test (and, in some cases, the first test) may be used to perform an operation with respect to the lead insertion procedure (e.g., provide a notification, provide feedback to a robotic lead insertion system, optimize parameters associated with the lead insertion procedure, etc.).

Compared with conventional approaches in which a single evoked response-based measurement test is used during a lead insertion procedure, the systems and methods described herein may optimize accuracy of electrode lead placement in the cochlea, improve efficiency of the lead insertion procedure in terms of computing resources used to monitor the lead insertion procedure, reduce time in which a cochlear implant recipient has to be in surgery, and/or provide various other technical and individual benefits and advantages, as will be made apparent herein.

As used herein, an evoked response may refer to any type of neural or physiological response within a cochlear implant recipient that occurs in response to any type of stimulation (e.g., acoustic and/or electrical stimulation). For example, an evoked response may include an electrocochleographic ("ECoG" or "ECochG") potential (e.g., a cochlear microphonic potential, an action potential, a summating potential, etc.), an auditory nerve response, a brainstem response, a compound action potential, a stapedius reflex, and/or any other type of neural or physiological response that may occur within a recipient in response to application of stimulation (e.g., acoustic and/or electrical stimulation) to the recipient. Evoked responses may originate from neural tissues, hair cell to neural synapses, inner or outer hair cells, and/or other sources.

An evoked response-based measurement test may refer to any test that may be performed that involves measuring at least one type of evoked response. For example, an evoked response-based measurement test may include a single tone-based insertion depth monitoring procedure that measures an evoked response (e.g., ECochG) that occurs in response to application of acoustic stimulation having a single frequency to determine an insertion depth of an electrode lead into the cochlea. As another example, an evoked response-based measurement test may include a multi tone-based insertion depth monitoring procedure that measures multiple evoked responses (e.g., ECochG) that occur in response to application of acoustic stimulation having multiple frequencies to determine an insertion depth of an electrode lead into the cochlea. As another example, an evoked response-based measurement test may include an electric field imaging (EFI) measurement procedure configured to determine a distance of the electrode lead from the modiolus and/or whether tip fold over has occurred with respect to the electrode lead. As another example, an evoked response-based measurement test may include an excitation spread measurement procedure configured to determine an extent to which electrical stimulation (e.g., an electrical pulse) applied by one electrode at one location may spread or travel (e.g., through fluid and/or tissue at and surrounding the location) so as to be detectable (e.g., as a voltage) by another electrode at another location. Any other type of evoked response-based measurement test may be used in accordance with the systems and methods described herein.

Signals representative of evoked responses may be measured in any suitable manner. For example, as described herein, a diagnostic system may direct an acoustic stimulation generator to apply acoustic stimulation having one or more stimulus frequencies to a recipient of a cochlear implant during an insertion procedure in which an electrode lead communicatively coupled to the cochlear implant is inserted into a cochlea of the recipient. The diagnostic system may direct the cochlear implant to use an electrode disposed on the electrode lead to record one or more evoked response signals during the insertion procedure. In some examples, each evoked response signal included in the one or more evoked response signals may correspond to a different stimulus frequency included in a plurality of stimulus frequencies and may be representative of one or more evoked responses that occur within the recipient in response to the acoustic stimulation applied to the recipient.

As described herein, attributes associated with the evoked response signals recorded by the electrode may be indicative of an insertion state of the electrode lead within the cochlea of the recipient. For example, an amplitude and/or a phase of one or more evoked response signals may be indicative of a particular insertion state. As used herein, "an insertion state" may correspond to any of a plurality of different insertion states that may be associated with insertion of the electrode lead into the cochlea of the recipient. For example, one or more insertion states may be associated with the electrode lead passing a characteristic frequency location of the cochlea, passing a cluster of hair cells or neurons, contacting a structure of the cochlea (e.g., the basilar membrane), causing trauma to the cochlea (e.g., passing through the basilar membrane), experiencing tip fold over, etc. Accordingly, in some examples, the diagnostic system may determine an insertion state of the electrode lead within the cochlea of the recipient based on an amplitude and/or a phase of each of one or more evoked response signals included in the plurality of evoked response signals. Other types of evoked response-based measurement tests, such as electric field imaging (EFI), may be used as may serve a particular implementation. An EFI measurement may include stimulation (including stimulation ground) and recording (including recording ground) from either same electrodes or different electrodes.

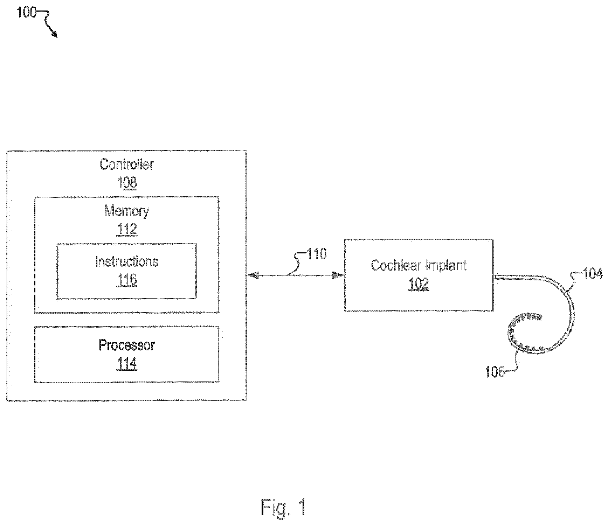

Fig. 1 shows an illustrative cochlear implant system 100 configured to be used by a recipient. As shown, cochlear implant system 100 includes a cochlear implant 102, an electrode lead 104 physically coupled to cochlear implant 102 and having an array of electrodes 106, and a controller 10 configured to be communicatively coupled to cochlear implant 102 by way of a communication link 110.

The cochlear implant system 100 shown in Fig. 1 is unilateral (i.e., associated with only one ear of the recipient). Alternatively, a bilateral configuration of cochlear implant system 100 may include separate cochlear implants and electrode leads for each ear of the recipient. In the bilateral configuration, controller 108 may be implemented by a single controller configured to interface with both cochlear implants or by two separate controllers each configured to interface with a different one of the cochlear implants.

Cochlear implant 102 may be implemented by any suitable type of implantable stimulator. For example, cochlear implant 102 may be implemented by an implantable cochlear stimulator. Additionally or alternatively, cochlear implant 102 may be implemented by a brainstem implant and/or any other type of device that may be implanted within the recipient and configured to apply electrical stimulation to one or more stimulation sites located along an auditory pathway of the recipient.

In some examples, cochlear implant 102 may be configured to generate electrical stimulation representative of an audio signal processed by controller 108 in accordance with one or more stimulation parameters transmitted to cochlear implant 102 by controller 108. Cochlear implant 102 may be further configured to apply the electrical stimulation to one or more stimulation sites (e.g., one or more intracochlear locations) within the recipient by way of one or more electrodes 106 on electrode lead 104. In some examples, cochlear implant 102 may include a plurality of independent current sources each associated with a channel defined by one or more of electrodes 106. In this manner, different stimulation current levels may be applied to multiple stimulation sites simultaneously by way of multiple electrodes 106.

Cochlear implant 102 may additionally or alternatively be configured to generate, store, and/or transmit data. For example, cochlear implant may use one or more electrodes 106 to record one or more signals (e.g., one or more voltages, impedances, evoked responses within the recipient, and/or other measurements) and transmit, by way of communication link 110, data representative of the one or more signals to controller 108. In some examples, this data is referred to as back telemetry data.

Electrode lead 104 may be implemented in any suitable manner. For example, a distal portion of electrode lead 104 may be pre-curved such that electrode lead 104 conforms with the helical shape of the cochlea after being implanted. Electrode lead 104 may alternatively be naturally straight or of any other suitable configuration.

In some examples, electrode lead 104 includes a plurality of wires (e.g., within an outer sheath) that conductively couple electrodes 106 to one or more current sources within cochlear implant 102. For example, if there are n electrodes 106 on electrode lead 104 and n current sources within cochlear implant 102, there may be n separate wires within electrode lead 104 that are configured to conductively connect each electrode 106 to a different one of the n current sources. Example values for n are 8, 12, 16, or any other suitable number.

Electrodes 106 are located on at least a distal portion of electrode lead 104. In this configuration, after the distal portion of electrode lead 104 is inserted into the cochlea, electrical stimulation may be applied by way of one or more of electrodes 106 to one or more intracochlear locations. One or more other electrodes (e.g., including a ground electrode, not explicitly shown) may also be disposed on other parts of electrode lead 104 (e.g., on a proximal portion of electrode lead 104) to, for example, provide a current return path for stimulation current applied by electrodes 106 and to remain external to the cochlea after the distal portion of electrode lead 104 is inserted into the cochlea. Additionally or alternatively, a housing of cochlear implant 102 may serve as a ground electrode for stimulation current applied by electrodes 106.

Controller 108 may be configured to interface with (e.g., control and/or receive data from) cochlear implant 102. For example, controller 108 may transmit commands (e.g., stimulation parameters and/or other types of operating parameters in the form of data words included in a forward telemetry sequence) to cochlear implant 102 by way of communication link 110. Controller 108 may additionally or alternatively provide operating power to cochlear implant 102 by transmitting one or more power signals to cochlear implant 102 by way of communication link 110. Controller 108 may additionally or alternatively receive data from cochlear implant 102 by way of communication link 110. Communication link 110 may be implemented by any suitable number of wired and/or wireless bidirectional and/or unidirectional links.

As shown, controller 108 includes a memory 112 and a processor 114 configured to be selectively and communicatively coupled to one another. In some examples, memory 112 and processor 114 may be distributed between multiple devices and/or multiple locations as may serve a particular implementation.

Memory 112 may be implemented by any suitable non-transitory computer-readable medium and/or non-transitory processor-readable medium, such as any combination of non-volatile storage media and/or volatile storage media. Illustrative non-volatile storage media include, but are not limited to, read-only memory, flash memory, a solid-state drive, a magnetic storage device (e.g., a hard drive), ferroelectric random-access memory ("RAM"), and an optical disc. Illustrative volatile storage media include, but are not limited to, RAM (e.g., dynamic RAM).

Memory 112 may maintain (e.g., store) executable data used by processor 114 to perform one or more of the operations described herein. For example, memory 112 may store instructions 116 that may be executed by processor 114 to perform any of the operations described herein. Instructions 116 may be implemented by any suitable application, program (e.g., sound processing program), software, code, and/or other executable data instance. Memory 112 may also maintain any data received, generated, managed, used, and/or transmitted by processor 114.

Processor 114 may be configured to perform (e.g., execute instructions 116 stored in memory 112 to perform) various operations with respect to cochlear implant 102.

To illustrate, processor 114 may be configured to control an operation of cochlear implant 102. For example, processor 114 may receive an audio signal (e.g., by way of a microphone communicatively coupled to controller 108, a wireless interface (e.g., a Bluetooth interface), and/or a wired interface (e.g., an auxiliary input port)). Processor 114 may process the audio signal in accordance with a sound processing program (e.g., a sound processing program stored in memory 112) to generate appropriate stimulation parameters. Processor 114 may then transmit the stimulation parameters to cochlear implant 102 to direct cochlear implant 102 to apply electrical stimulation representative of the audio signal to the recipient.

In some implementations, processor 114 may also be configured to apply acoustic stimulation to the recipient. For example, a receiver (also referred to as a loudspeaker) may be optionally coupled to controller 108. In this configuration, processor 114 may deliver acoustic stimulation to the recipient by way of the receiver. The acoustic stimulation may be representative of an audio signal (e.g., an amplified version of the audio signal), configured to elicit an evoked response within the recipient, and/or otherwise configured. In configurations in which processor 114 is configured to both deliver acoustic stimulation to the recipient and direct cochlear implant 102 to apply electrical stimulation to the recipient, cochlear implant system 100 may be referred to as a bimodal hearing system and/or any other suitable term.

Processor 114 may be additionally or alternatively configured to receive and process data generated by cochlear implant 102. For example, processor 114 may receive data representative of a signal recorded by cochlear implant 102 using one or more electrodes 106 and, based on the data, adjust one or more operating parameters of controller 108. Additionally or alternatively, processor 114 may use the data to perform one or more diagnostic operations with respect to cochlear implant 102 and/or the recipient.

Other operations may be performed by processor 114 as may serve a particular implementation. In the description provided herein, any references to operations performed by controller 108 and/or any implementation thereof may be understood to be performed by processor 114 based on instructions 116 stored in memory 112.

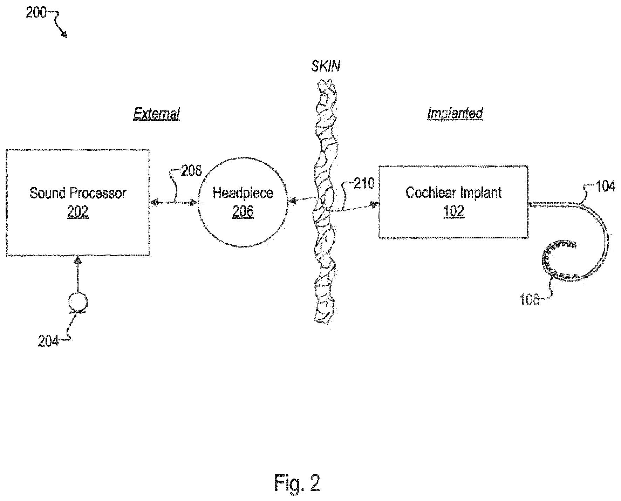

Controller 108 may be implemented by one or more devices configured to interface with cochlear implant 102. To illustrate, Fig. 2 shows an illustrative configuration 200 of cochlear implant system 100 in which controller 108 is implemented by a sound processor 202 configured to be located external to the recipient. In configuration 200, sound processor 202 is communicatively coupled to a microphone 204 and to a headpiece 206 that are both configured to be located external to the recipient.

Sound processor 202 may be implemented by any suitable device that may be worn or carried by the recipient. For example, sound processor 202 may be implemented by a behind-the-ear ("BTE") unit configured to be worn behind and/or on top of an ear of the recipient. Additionally or alternatively, sound processor 202 may be implemented by an off-the-ear unit (also referred to as a body worn device) configured to be worn or carried by the recipient away from the ear. Additionally or alternatively, at least a portion of sound processor 202 is implemented by circuitry within headpiece 206.

Microphone 204 is configured to detect one or more audio signals (e.g., that include speech and/or any other type of sound) in an environment of the recipient. Microphone 204 may be implemented in any suitable manner. For example, microphone 204 may be implemented by a microphone that is configured to be placed within the concha of the ear near the entrance to the ear canal, such as a T-MIC^{™} microphone from Advanced Bionics. Such a microphone may be held within the concha of the ear near the entrance of the ear canal during normal operation by a boom or stalk that is attached to an ear hook configured to be selectively attached to sound processor 202. Additionally or alternatively, microphone 204 may be implemented by one or more microphones in or on headpiece 206, one or more microphones in or on a housing of sound processor 202, one or more beam-forming microphones, and/or any other suitable microphone as may serve a particular implementation.

Headpiece 206 may be selectively and communicatively coupled to sound processor 202 by way of a communication link 208 (e.g., a cable or any other suitable wired or wireless communication link), which may be implemented in any suitable manner. Headpiece 206 may include an external antenna (e.g., a coil and/or one or more wireless communication components) configured to facilitate selective wireless coupling of sound processor 202 to cochlear implant 102. Headpiece 206 may additionally or alternatively be used to selectively and wirelessly couple any other external device to cochlear implant 102. To this end, headpiece 206 may be configured to be affixed to the recipient's head and positioned such that the external antenna housed within headpiece 206 is communicatively coupled to a corresponding implantable antenna (which may also be implemented by a coil and/or one or more wireless communication components) included within or otherwise connected to cochlear implant 102. In this manner, stimulation parameters and/or power signals may be wirelessly and transcutaneously transmitted between sound processor 202 and cochlear implant 102 by way of a wireless communication link 210.

In configuration 200, sound processor 202 may receive an audio signal detected by microphone 204 by receiving a signal (e.g., an electrical signal) representative of the audio signal from microphone 204. Sound processor 202 may additionally or alternatively receive the audio signal by way of any other suitable interface as described herein. Sound processor 202 may process the audio signal in any of the ways described herein and transmit, by way of headpiece 206, stimulation parameters to cochlear implant 102 to direct cochlear implant 102 to apply electrical stimulation representative of the audio signal to the recipient.

In an alternative configuration, sound processor 202 may be implanted within the recipient instead of being located external to the recipient. In this alternative configuration, which may be referred to as a fully implantable configuration of cochlear implant system 100, sound processor 202 and cochlear implant 102 may be combined into a single device or implemented as separate devices configured to communicate one with another by way of a wired and/or wireless communication link. In a fully implantable implementation of cochlear implant system 100, headpiece 206 may not be included and microphone 204 may be implemented by one or more microphones implanted within the recipient, located within an ear canal of the recipient, and/or external to the recipient.

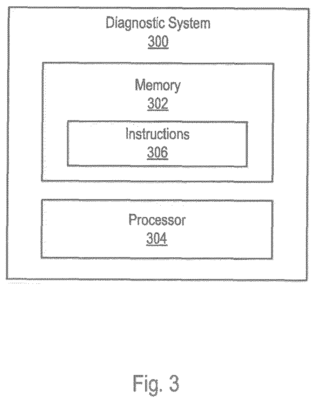

Fig. 3 shows an illustrative diagnostic system 300 that may be configured to perform any of the operations described herein. As shown, diagnostic system 300 may include, without limitation, a storage facility 302 and a processing facility 304 selectively and communicatively coupled to one another. Facilities 302 and 304 may each include or be implemented by hardware and/or software components (e.g., processors, memories, communication interfaces, instructions stored in memory for execution by the processors, etc.). In some examples, facilities 302 and 304 may be distributed between multiple devices and/or multiple locations as may serve a particular implementation.

Storage facility 302 may maintain (e.g., store) executable data used by processing facility 304 to perform any of the operations described herein. For example, storage facility 302 may store instructions 306 that may be executed by processing facility 304 to perform any of the operations described herein. Instructions 306 may be implemented by any suitable application, software, code, and/or other executable data instance. Storage facility 302 may also maintain any data received, generated, managed, used, and/or transmitted by processing facility 304.

Processing facility 304 may be configured to perform (e.g., execute instructions 306 stored in storage facility 302 to perform) various operations. For example, processing facility 304 may direct an acoustic stimulation generator to apply acoustic stimulation having a plurality of stimulus frequencies to a recipient of a cochlear implant during an insertion procedure in which an electrode lead communicatively coupled to the cochlear implant is inserted into a cochlea of the recipient, direct the cochlear implant to use an electrode disposed on the electrode lead to record a plurality of evoked response signals during the insertion procedure, each evoked response signal included in the plurality of evoked response signals corresponding to a different stimulus frequency included in the plurality of stimulus frequencies and representative of evoked responses that occur within the recipient in response to the acoustic stimulation applied to the recipient, and determine, based on an amplitude and a phase of each of one or more evoked response signals included in the plurality of evoked response signals, an insertion state of the electrode lead within the cochlea of the recipient. These and other operations that may be performed by processing facility 304 are described in more detail herein.

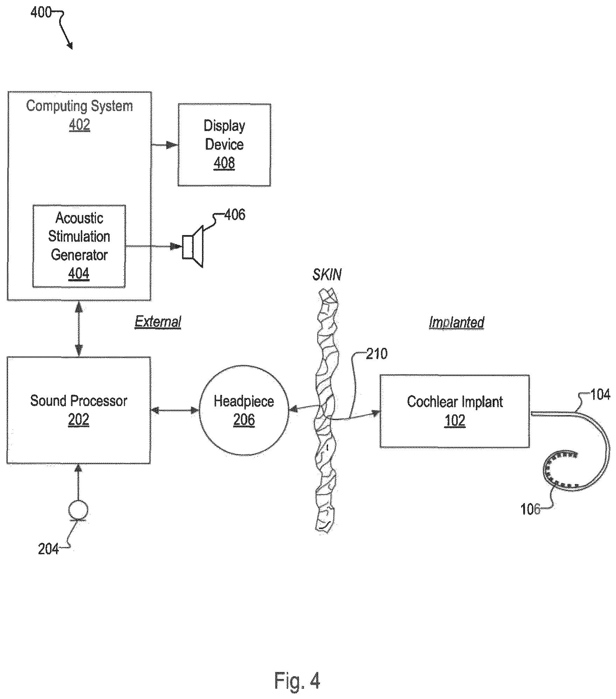

Diagnostic system 300 may be implemented in any suitable manner. For example, Fig. 4 shows an illustrative configuration 400 in which diagnostic system 300 is implemented by a computing system 402 configured to communicatively couple to sound processor 202. As shown, computing system 402 may include an acoustic stimulation generator 404 communicatively coupled to a speaker 406. Computing system 402 is also communicatively coupled to a display device 408. While computing system 402 is described herein as being be coupled to sound processor 202, computing system 402 may be alternatively coupled to any other implementation of controller 108 as may serve a particular implementation.

Computing system 402 may be implemented by any suitable combination of hardware (e.g., one or more computing devices) and software. For example, computing system 402 may be implemented by a computing device programmed to perform one or more fitting operations with respect to a recipient of a cochlear implant. To illustrate, computing system 402 may be implemented by a desktop computer, a mobile device (e.g., a laptop, a smartphone, a tablet computer, etc.), and/or any other suitable computing device as may serve a particular implementation. As an example, computing system 402 may be implemented by a mobile device configured to execute an application (e.g., a "mobile app") that may be used by a user (e.g., the recipient, a clinician, and/or any other user) to control one or more settings of sound processor 202 and/or cochlear implant 102 and/or perform one or more operations (e.g., diagnostic operations) with respect to data generated by sound processor 202 and/or cochlear implant 102.

Acoustic stimulation generator 404 may be implemented by any suitable combination of components configured to generate acoustic stimulation. In some examples, the acoustic stimulation may include one or more tones having one or more stimulus frequencies. Additionally or alternatively, the acoustic stimulation may include any other type of acoustic content that has at least a particular stimulus frequency of interest. Speaker 406 may be configured to deliver the acoustic stimulation generated by acoustic stimulation generator 404 to the recipient. For example, speaker 406 may be implemented by an ear mold configured to be placed in or near an entrance to an ear canal of the recipient.

Display device 408 may be implemented by any suitable device configured to display graphical content generated by computing system 402. For example, display device 408 may display one or more graphs of evoked responses recorded by an electrode disposed on electrode lead 104. Display device 408 is shown in Fig. 4 as an external device configured to display content generated by computing system 402. Additionally or alternatively, computing system 402 may include display device 408 as an integrated display in certain implementations.

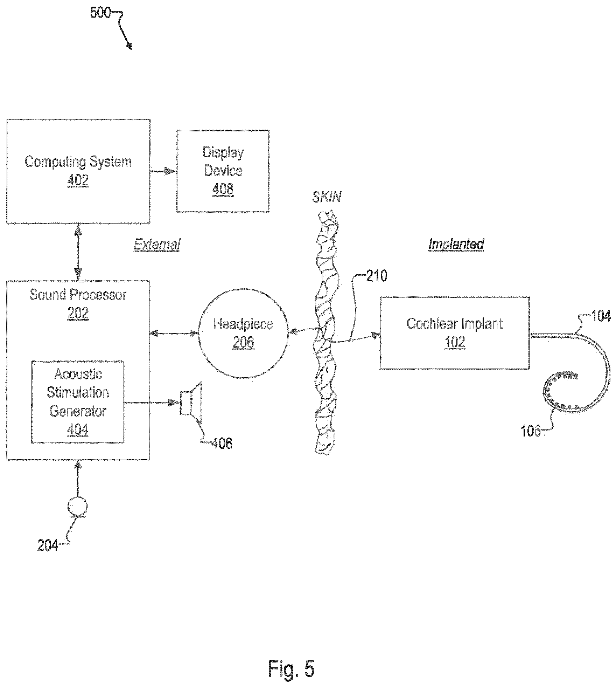

Fig. 5 shows another exemplary configuration 500 in which diagnostic system 300 is implemented by computing system 402. In configuration 500, acoustic stimulation generator 404 is included in sound processor 202. For example, sound processor 202 may be implemented by a bimodal sound processor (i.e., a sound processor configured to direct cochlear implant 102 to apply electrical stimulation to a recipient and acoustic stimulation generator 404 to apply acoustic stimulation to the recipient). In some examples, speaker 406 may be implemented by an audio ear hook that connects to sound processor 202.

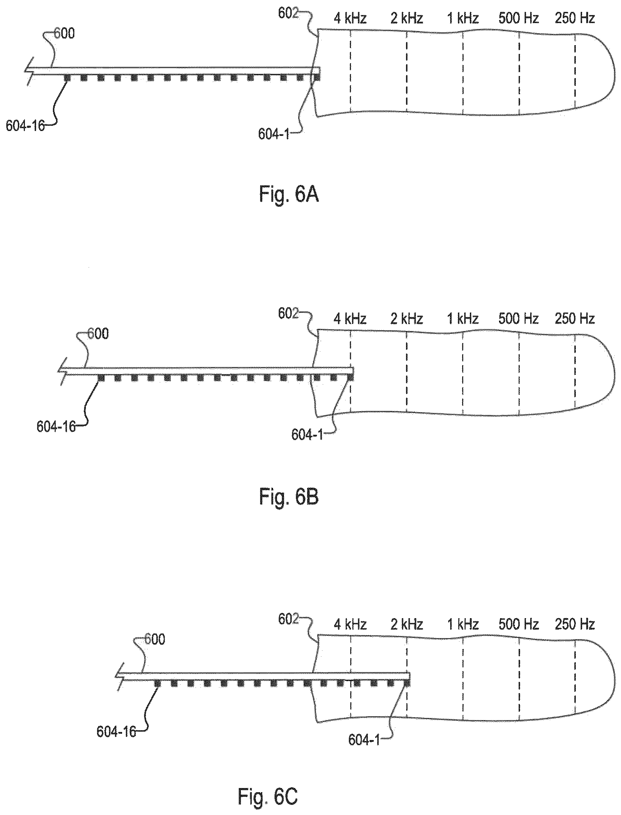

Figs. 6A-6F illustrate an exemplary insertion procedure in which an electrode lead 600 is inserted into a cochlea 602 of a recipient. For illustrative purposes, cochlea 602 is depicted in Figs. 6A-6F as being "unrolled" instead of its actual curved, spiral shape. Electrode lead 600 may be similar to electrode lead 104 and may include a plurality of electrodes (e.g., electrodes 604-1 through electrode 604-16) disposed thereon. Electrode 604-1 is a distal-most electrode on electrode lead 600 and electrode 604-16 is a proximal-most electrode on electrode lead 600.

Various characteristic frequency locations within cochlea 602 are depicted by vertical dashed lines in each of Figs. 6A-6F. As shown, a first characteristic frequency location is associated with 4 kHz. Hence, electrical stimulation applied by an electrode positioned at this characteristic frequency location may result in the recipient perceiving sound having 4 kHz or the hair cell and neural structures there respond to 4 kHz acoustic stimulus. Figs. 6A-6F also depict characteristic frequency locations associated with 2 kHz, 1 kHz, 500 Hz, and 250 Hz. As shown, the frequencies associated with the characteristic frequency locations are tonotopically arranged, with relatively higher frequencies being located towards the entrance (or base) of cochlea 602 and relatively lower frequencies being located towards the distal end (or apex) of cochlea 602.

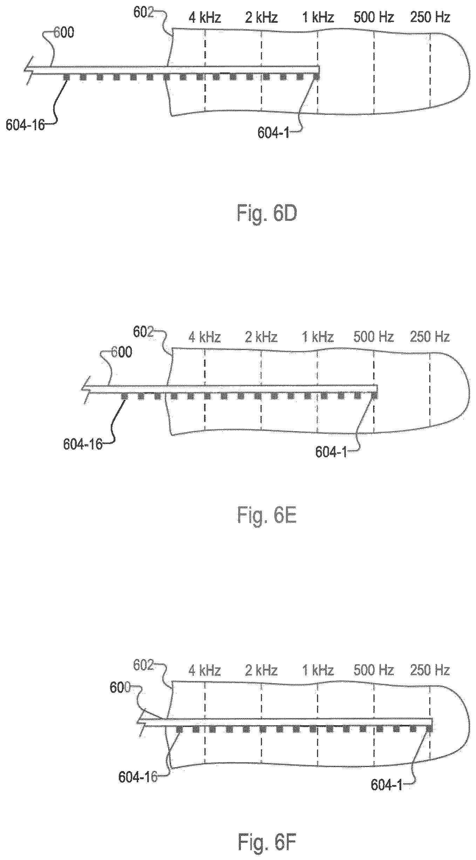

Fig. 6A shows electrode lead 600 entering cochlea 602. In this figure, electrode 604-1 is barely within cochlea 602. Fig. 6B shows electrode lead 600 after electrode lead 600 has been advanced further into cochlea 602 such that electrode 604-1 is positioned at the characteristic frequency location corresponding to 4 kHz. Figs. 6C-6F show electrode lead 600 after electrode lead 600 has been advanced further into cochlea 602 such that electrode 604-1 is positioned at the characteristic frequency location corresponding to 2 kHz (Fig. 6C), then 1 kHz (Fig. 6D), then 500 Hz (Fig. 6E), and then 250 Hz (Fig. 6F).

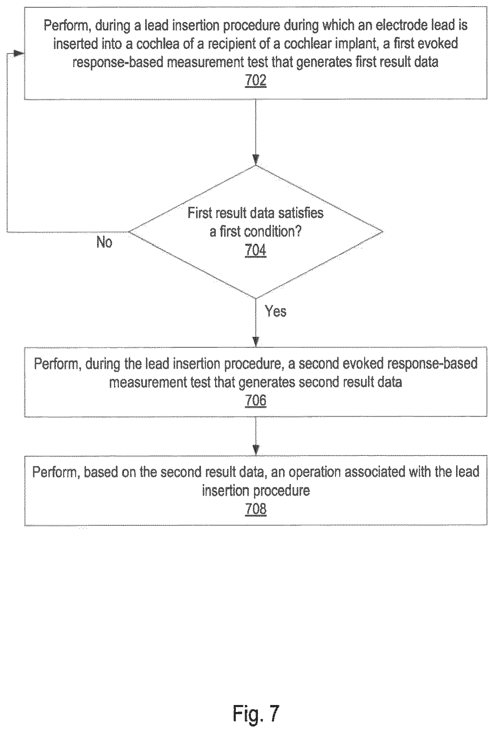

Fig. 7 illustrates an illustrative method 700 that may be used to perform state-based initiation of evoked response-based measurement tests during an electrode lead insertion procedure. The operations shown in Fig. 7 may be performed by diagnostic system 300 and/or any implementation thereof. While Fig. 7 illustrates exemplary operations according to one embodiment, other embodiments may omit, add to, reorder, and/or modify any of the operations shown in Fig. 7.

At operation 702, diagnostic system 300 performs, during a lead insertion procedure during which an electrode lead is inserted into a cochlea of a recipient of a cochlear implant, a first evoked response-based measurement test that generates first result data. The first evoked response-based measurement test may be any of the evoked response-based measurement tests described herein. For example, the first evoked response-based measurement test may be a single tone-based insertion depth monitoring procedure, a multi tone-based insertion depth monitoring procedure, an EFI measurement procedure, an excitation spread measurement procedure, and/or any other type of evoked response-based measurement test as may serve a particular implementation.

As mentioned, the first evoked response-based measurement test is performed during a lead insertion procedure. As used herein, a lead insertion procedure commences when the electrode lead is introduced into a recipient. The lead insertion procedure ends when the electrode lead is located at a final location within the cochlea or when the electrode lead is completely retracted or removed from the recipient (e.g., which may happen if trauma to the cochlea occurs and the surgeon decides that it is better to completely remove the electrode lead from the recipient). Hence, "during" a lead insertion procedure refers to any time between commencement of the lead insertion procedure and when the lead insertion procedure ends. It will be recognized that actual insertion of the electrode lead may be paused at any time during the lead insertion procedure and that any of the evoked response-based measurement tests may be performed while such insertion is paused.

At decision 704, diagnostic system 300 determines whether the first result data satisfies a first condition. For example, diagnostic system 300 may determine whether the first result data indicates that a detected evoked response changes in amplitude and/or phase by more than a threshold amount. These and various other conditions that the first result data may satisfy are described herein.

If the first result data does not satisfy the first condition (No, decision 704), diagnostic system 300 continues performing the first evoked response-based measurement test at operation 702.

Alternatively, if the first result data does satisfy the first condition (Yes, decision 704), diagnostic system performs, during the lead insertion procedure, a second evoked response-based measurement test that generates second result data (operation 706). The second evoked response-based measurement test is of a different type than the first evoked response-based measurement test. For example, if the first evoked response-based measurement test is a single tone-based insertion depth monitoring procedure, the second evoked response-based measurement test may be any evoked response-based measurement test other than a single tone-based insertion depth monitoring procedure (e.g., a multi tone-based insertion depth monitoring procedure, an EFI measurement procedure, or an excitation spread measurement procedure).

In some examples, diagnostic system 300 automatically initiates performance of the second evoked response-based measurement test in place of the first evoked response-based measurement test without user input being provided to initiate the second evoked response-based measurement test. Alternatively, diagnostic system 300 may provide a notification that the first condition has been satisfied. The user may then manually initiate the second evoked response-based measurement test.

In some examples, diagnostic system 300 may select, based on the first result data, a particular evoked response-based measurement test from a plurality of available evoked response-based measurement tests and designate the particular evoked response-based measurement test as the second evoked response-based measurement test. For example, the first result data may indicate that a particular evoked response-based measurement test would be better suited to confirm the first result data than other evoked response-based measurement tests available for use. Based on this, diagnostic system 300 may use the particular evoked response-based measurement test as the second evoked response-based measurement test performed at operation 706.

In some examples, the selection of the particular evoked response-based measurement test for use at operation 706 may be based on an output of a machine learning model. For example, diagnostic system 300 may maintain or otherwise access data representative of a machine learning model. The machine learning model may be configured to perform any suitable machine learning heuristic (also referred to as artificial intelligence heuristic) with respect to first result data. The machine learning model may accordingly be supervised and/or unsupervised as may serve a particular implementation and may be configured to implement one or more decision tree learning algorithms, association rule learning algorithms, artificial neural network learning algorithms, deep learning algorithms, bitmap algorithms, and/or any other suitable data analysis technique as may serve a particular implementation.

In some examples, the machine learning model is implemented by one or more neural networks, such as one or more deep convolutional neural networks (CNN) using internal memories of its respective kernels (filters), recurrent neural networks (RNN), and/or long/short term memory neural networks (LSTM). The machine learning model may be multi-layer. For example, the machine learning model may be implemented by a neural network that includes an input layer, one or more hidden layers, and an output layer.

The machine learning model may be trained in any suitable manner. For example, diagnostic system 300 may provide historical test result data (e.g., data associated with one or more lead insertion procedures performed prior to the lead insertion procedure with respect to the patient and/or other patients) as a training input to machine learning model. The machine learning model may accordingly be trained to select an optimal second evoked response-based measurement test at operation 706.

At operation 708, diagnostic system 300 performs, based on the second result data, an operation associated with the lead insertion procedure. The operation may be any suitable operation, examples of which are described herein.

To illustrate, the operation may include providing, during the lead insertion procedure, a notification (e.g., a notification indicative of a particular insertion state of the electrode lead). The notification may be a visual and/or audio notification (e.g., a graphic displayed on a display screen that is viewable by surgical personnel performing the lead insertion procedure and/or an audible sound presented by way of a speaker within a vicinity of the surgical personnel). For example, the notification may indicate a particular insertion state (e.g., that the electrode lead has been inserted a particular distance within the cochlea, that the electrode lead has been inadvertently caused damage to the cochlea, etc.).

The operation may additionally or alternatively include adjusting one or more parameters configured to control the lead insertion procedure. For example, the operation may include providing feedback to a robotic lead insertion system. The robotic lead insertion system may be configured to perform the lead insertion procedure in accordance with one or more operating parameters. In this example, the feedback may be in the form of one or more signals configured to adjust one or more of the one or more operating parameters. This may be performed automatically without user intervention. Alternatively, a user may provide user input that approves the feedback before the one or more operating parameters are adjusted.

The operation may additionally or alternatively include providing feedback (e.g., through audio or visual alarms) to a user (e.g., a surgeon or other user), the feedback configured to direct the user to change an insertion angle, retract the electrode lead, change a speed at which the electrode lead is inserted, and/or adjust any other aspect of the lead insertion procedure.

The operation may additionally or alternatively include determining that the second result data satisfies a second condition. Based on this determination, diagnostic system 300 may perform a third evoked response-based measurement test that generates third result data. The third evoked response-based measurement test may be of a different type than the second evoked response-based measurement test (and, in some examples, the first evoked response-based measurement test). In this example, the operation performed at 708 may be further based on the third result data.

As mentioned, the operation performed at operation 708 may be based on the second result data generated by the second evoked response-based measurement test performed at operation 706. In some examples, operation 708 is further based on first result data generated by the first evoked response-based measurement test performed at operation 702.

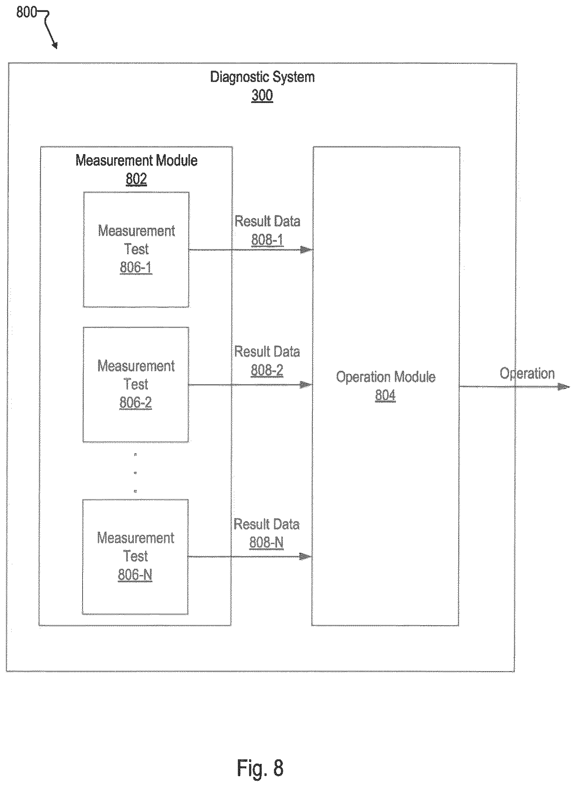

Fig. 8 shows an implementation 800 of diagnostic system 300 configured to implement the method 700 of Fig. 7. As shown, diagnostic system 300 may include a measurement module 802 and an operation module 804. Measurement module 802 may be configured to selectively perform a plurality of different evoked response-based measurement tests 806-1 through 806-N. Each evoked response-based measurement test may generate result data 808 (e.g., result data 808-1 through result data 808-N). As shown, result data 808 may be provided as inputs to operation module 804, which may perform an operation based on one or more of result data 808.

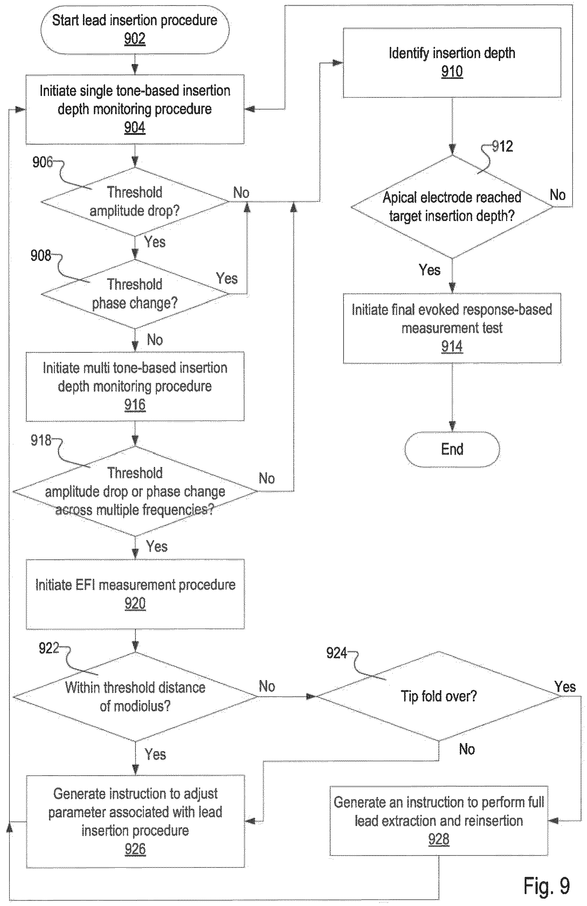

A specific example of the methods described herein is illustrated in Fig. 9. The operations shown in Fig. 9 may be performed by diagnostic system 300 and/or any implementation thereof. While Fig. 9 illustrates exemplary operations according to one embodiment, other embodiments may omit, add to, reorder, and/or modify any of the operations shown in Fig. 9.

A lead insertion procedure starts at operation 902. During the lead insertion procedure, a user (e.g., a surgeon) and/or a robotic insertion system inserts an electrode lead into a cochlea of a recipient.

During the lead insertion procedure, diagnostic system 300 initiates a single tone-based insertion depth monitoring procedure at operation 904. As described herein, the single tone-based insertion depth monitoring procedure may include applying an acoustic stimulation having a single tone or frequency and monitoring an evoked response (e.g., an ECochG) with an electrode (e.g., the most apical electrode) on the electrode lead. Result data generated by the single tone-based insertion depth monitoring procedure may be representative of an evoked response signal recorded by the electrode.

Decisions 906 and 908 may determine whether the result data generated by the single tone-based insertion depth monitoring procedure satisfies one or more conditions. In particular, at decision 906, diagnostic system 300 determines whether there is a threshold amplitude drop in the evoked response. In other words, diagnostic system 300 determines whether an amplitude of the evoked response signal decreases by more than a threshold amount during a particular amount of time.

If there is not a threshold amplitude drop (No, decision 906), diagnostic system 901 identifies an insertion depth (e.g., how many electrodes have been inserted into the cochlea) based on the result data generated by the single tone-based insertion depth monitoring procedure (operation 910). This determination can be made in any suitable manner based on the evoked response signal recorded by the electrode.

At decision 912, diagnostic system 300 determines whether the most apical electrode has reached a target insertion depth. This determination may be made in any suitable manner. If the target insertion depth has not been reached (No, decision 912), diagnostic system 300 continues with the single tone-based insertion depth monitoring procedure at operation 904.

If the target insertion depth has been reached (Yes, decision 912), diagnostic system 300 initiates a final evoked response-based measurement test at operation 914. This final evoked response-based measurement test may include any of the evoked response-based measurement tests described herein, such as an EFI test to determine electrode state. At operation 914, diagnostic system 300 may additionally perform any other type of test, such as a post-operative audiogram to determine a mapping between frequencies and electrode locations.

Returning to decision 906, if a threshold amplitude drop is detected (Yes, decision 906), diagnostic system 300 determines whether there is a threshold phase change in the evoked response signal at decision 908. In other words, diagnostic system 300 determines whether a phase of the evoked response signal changes by more than a threshold amount during a particular amount of time.

If a threshold phase change is detected (Yes, decision 908), the method proceeds to operation 910 (identification of the insertion depth, as described above). If a threshold phase change is not detected (No, decision 908), diagnostic system 300 initiates a multi tone-based insertion depth monitoring procedure at operation 916. As described herein, the multi tone-based insertion depth monitoring procedure may include applying an acoustic stimulation having a plurality of tones or frequencies and monitoring a plurality of evoked response signals with one or more electrodes (e.g., the most apical electrodes) on the electrode lead. Result data generated by the multi tone-based insertion depth monitoring procedure may be representative of one or more evoked response signals recorded by the one or more electrodes.

The multi tone-based insertion depth monitoring procedure may provide more accurate result data than the single tone-based insertion depth monitoring procedure and/or confirm the result data generated by the single tone-based insertion depth monitoring procedure. By waiting to initiate the multi tone-based insertion depth monitoring procedure until a threshold amplitude drop is detected, but without a threshold phase change being detected, processing resources associated with the multi tone-based depth monitoring procedure may be conserved until a condition (i.e., the threshold amplitude drop) is detected using the single tone-based insertion depth monitoring procedure.

At decision 918, diagnostic system 300 determines, based on the one or more evoked response signals generated by the multi tone-based insertion depth monitoring procedure, whether there is a threshold amplitude drop and/or a threshold phase change across multiple frequencies. If not (No, decision 918), the method proceeds to operation 910 (identification of the insertion depth, as described above).

If there is a threshold amplitude drop and/or a threshold phase change across multiple frequencies (Yes, decision 918), diagnostic system 300 initiates an EFI measurement procedure at operation 920. Test result data generated by the EFI measurement procedure may be used to determine whether the electrode lead is within a threshold distance of the modiolus (decision 922) and/or if tip fold over has occurred with respect to the electrode lead (decision 924).

If the test result data generated by the EFI measurement procedure indicates that the electrode lead is within the threshold distance of the modiolus (Yes, decision 922), diagnostic system 300 generates an instruction to adjust a parameter associated with the lead insertion procedure at operation 926. The instruction may be provided to a user and/or a robotic insertion system. In some examples, this instruction may include instructing the user and/or the robotic insertion system to perform a minor extraction of the electrode lead and then proceed to insertion of the electrode lead again. The method then returns to operation 904.

If the test result data generated by the EFI measurement procedure indicates that the electrode lead is not within the threshold distance of the modiolus (No, decision 922) and that there is not tip fold over (No, decision 924), diagnostic system 300 generates the instruction at operation 926 to adjust a parameter associated with the lead insertion procedure. The method then returns to operation 904.

Alternatively, if diagnostic system 300 determines that there is tip fold over (Yes, decision 924), diagnostic system 300 generates an instruction to perform a full lead extraction and reinsertion at operation 928. The method then returns to operation 904.

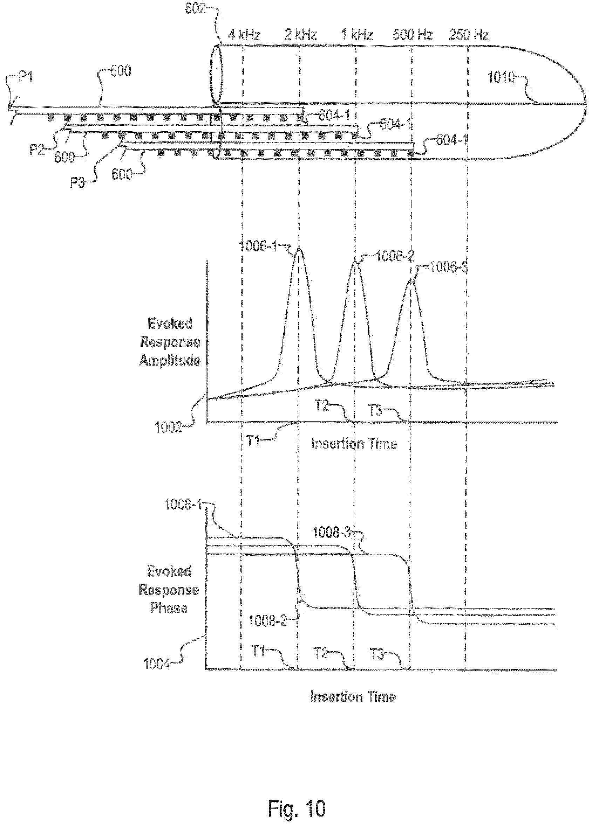

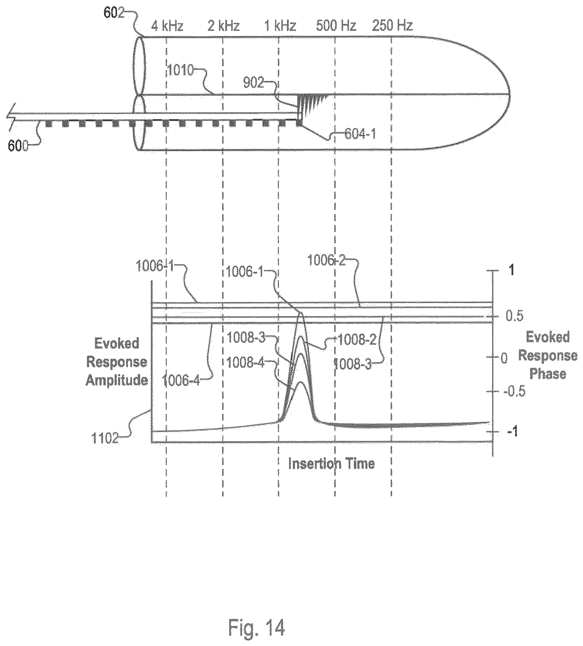

An illustrative multi tone-based insertion depth monitoring procedure and various conditions that may be detected using a multi-tone based insertion depth procedure will now be described in connection with Figs. 10-14. Result data generated by diagnostic system 300 while performing the multi tone-based insertion depth monitoring procedure may be indicative of any of these conditions. Multi tone-based insertion depth monitoring procedures are described in more detail in U.S. Patent Publication No. US20220233861A1, the contents of which are incorporated herein by reference. It will be recognized that a single tone-based insertion depth monitoring procedure may be similar to that described in connection with Figs. 10-14, except that only a single acoustic stimulation frequency is applied during a given time period.

In a multi tone-based insertion depth monitoring procedure, diagnostic system 300 may direct an acoustic stimulation generator (e.g., acoustic stimulation generator 404) to apply acoustic stimulation having a plurality of stimulus frequencies (i.e., concurrently) to a recipient of a cochlear implant during an insertion procedure in which an electrode lead communicatively coupled to the cochlear implant is inserted into a cochlea of the recipient. Diagnostic system 300 may direct the acoustic stimulation generator to apply the acoustic stimulation having the plurality of stimulus frequencies in any suitable manner. For example, diagnostic system 300 may direct the acoustic stimulation generator to continuously apply the acoustic stimulation during an insertion procedure, intermittently apply the acoustic stimulation during the insertion procedure, simultaneously apply different stimulus frequencies of the acoustic stimulation during the insertion procedure, sequentially apply the different stimulus frequencies of the acoustic stimulation during the insertion procedure, or apply the acoustic stimulation in any other suitable manner as may serve a particular implementation.

The acoustic stimulation may have any suitable plurality of stimulus frequencies as may serve a particular implementation. In certain examples, the acoustic stimulation may have four different stimulus frequencies that are concurrently applied during an insertion procedure. For example, in certain implementations the acoustic stimulation may include a first stimulus frequency corresponding to 2 kHz, a second stimulus frequency corresponding to 1 kHz, a third stimulus frequency corresponding to 500 Hz, and a fourth stimulus frequency corresponding to 250 Hz. In certain alternative implementations, the acoustic stimulation may have less than or more than four stimulus frequencies.

The acoustic stimulation is configured to produce a plurality of evoked responses during an insertion procedure that are useful in determining an insertion state. Accordingly, diagnostic system 300 may direct cochlear implant 102 to use an electrode to record a plurality of evoked response signals during an insertion procedure. Diagnostic system 300 may direct cochlear implant 102 to use any suitable electrode or combination of electrodes on an electrode lead to record the plurality of evoked response signals. For example, in certain implementations, diagnostic system 300 may direct the cochlear implant to use a distal-most electrode (e.g., electrode 604-1), also referred to as a most apical electrode, to record the plurality of evoked response signals. Each evoked response signal included in the plurality of evoked response signals may correspond to a different stimulus frequency included in the plurality of stimulus frequencies. In addition, each evoked response signal included in the plurality of evoked response signals may be representative of evoked responses that occur within the recipient in response to the acoustic stimulation applied to the recipient.

In certain examples, the plurality of evoked response signals may be considered as being included as part of a single evoked response detected by diagnostic system 300 in response to the acoustic stimulation applied to the recipient.

Attributes of the plurality of evoked response signals may be indicative of an insertion state of the electrode lead as the electrode lead is inserted within the cochlea. For example, as the electrode lead is inserted within the cochlea, an amplitude and/or a phase of one or more evoked response signals included in the plurality of evoked response signals may change in a manner that is indicative of a particular insertion state of the electrode lead. Accordingly, based on an amplitude and a phase of each of one or more evoked response signal included in the plurality of evoked response signals, diagnostic system 300 may determine an insertion state of the electrode lead within the cochlea of the recipient.

Diagnostic system 300 may determine any suitable number and/or type of insertion states as may serve a particular implementation. In certain examples, an insertion state may correspond to the electrode lead passing a particular characteristic frequency location within the cochlea. In such examples, diagnostic system 300 may determine that the electrode lead passes the particular characteristic frequency location when, within a predetermined amount of time, both an amplitude of a particular evoked response signal included in the plurality of evoked response signals decreases by at least an amplitude threshold amount and a phase of the particular evoked response signal changes by at least a phase threshold amount. The particular characteristic frequency location may correspond to a particular stimulus frequency that corresponds to the particular evoked response signal and that is included in the plurality of stimulus frequencies. Accordingly, diagnostic system 300 may determine an insertion state as passing a certain characteristic frequency location based on which evoked response signal has both a decrease in amplitude by at least an amplitude threshold amount and a phase change by at least a phase threshold amount.

To illustrate, Fig. 10 shows an exemplary lead insertion procedure in which electrode lead 600 is advanced into cochlea 602. Reference numbers P1 through P3 indicate positions of electrode lead 600. For example, at position P1, electrode lead 600 is at a first position in which electrode 604-1 is at the characteristic frequency location that corresponds to 2 kHz. At position P2, electrode lead 600 is at a second position in which electrode 604-1 is at the characteristic frequency location that corresponds to 1 kHz. At position P3, electrode lead 600 is at a third position in which electrode 604-1 is at the characteristic frequency location that corresponds to 500 Hz.

Fig. 10 also shows a graph 1002 of amplitudes 1006 (e.g., amplitudes 1006-1 through 1006-3) of evoked response signals recorded by electrode 604-1 at different insertion times T (e.g., T1 through T3) during the lead insertion procedure. In addition, Fig. 10 shows a graph 1004 of phases 1008 (e.g., phases 1008-1 through 1008-3) of the evoked response signals recorded by electrode 604-1 at different insertion times T during the lead insertion procedure. In this example, first, second, and third evoked response signals are generated in response to acoustic stimulation having stimulus frequencies of 2 kHz, 1 kHz, and 500 Hz, respectively. Hence, as shown in graph 1002, as electrode lead 600 advances towards the characteristic frequency location that corresponds to 2 kHz, the amplitude 1006-1 of a first evoked response signal, which is generated in response to acoustic stimulation having a stimulus frequencies of 2 kHz, increases and peaks at insertion time T1 when electrode lead 600 is positioned at position P1. As electrode lead 600 passes the characteristic frequency location that corresponds to 2 kHz, the first evoked response amplitude 1006-1 decreases until it settles at a steady state value. As shown in graph 1004, as electrode lead 600 advances towards the characteristic frequency location that corresponds to 2 kHz, the phase 1008-1 of the first evoked response signal remains at a relatively high level. However, the phase 1008-1 suddenly changes to a relatively low level at insertion time T1 as electrode lead 600 passes the characteristic frequency location that corresponds to 2 kHz.

As shown in Fig. 10, the decreasing of the first evoked response amplitude 1006-1 and the changing of the phase 1008-1 from the high level to the low level occur at substantially the same insertion time T1, and both occur as electrode 604-1 passes the characteristic frequency location that corresponds to 2 kHz. Hence, diagnostic system 300 may determine that electrode 604-1 passes the characteristic frequency location that corresponds to 2 kHz by detecting, within a predetermined time period, that both an amplitude 1006-1 of the first evoked response signal recorded by electrode 604-1 decreases by at least an amplitude threshold amount and a phase 1008-1 of the first evoked response signal recorded by electrode 604-1 changes by at least a phase threshold amount. The predetermined time period, the amplitude threshold amount, and/or the phase threshold amount may each be set by diagnostic system 300 to be any suitable value. For example, the predetermined time period may be set to be a relatively short time period (e.g., less than a few milliseconds) to ensure that the change in amplitude and in phase correspond to one another. In some examples, diagnostic system 300 may set the predetermined time period, the amplitude threshold amount, and/or the phase threshold in response to user input (e.g., by way of a graphical user interface). Additionally or alternatively, diagnostic system 300 may set the predetermined time period, the amplitude threshold amount, and/or the phase threshold automatically based on one or more factors, such as hearing loss, the stimulus frequency, recipient characteristics (e.g., age, gender, etc.), etc.

As is further shown in Fig. 10, after electrode lead 600 passes the characteristic frequency location that corresponds to 2 kHz, electrode lead 600 advances towards the characteristic frequency location that corresponds to 1 kHz. As electrode lead 600 advances toward the characteristic frequency location that corresponds to 1 kHz, the amplitude 1006-2 of the second evoked response signal, which is generated in response to acoustic stimulation having a stimulus frequencies of 1 kHz, increases and peaks at insertion time T2 when electrode lead 600 is positioned at position P2. As electrode lead 600 passes the characteristic frequency location that corresponds to 1 kHz, the second evoked response amplitude 1006-2 decreases until it settles at a steady state value. As shown in graph 1004, as electrode lead 600 advances towards the characteristic frequency location that corresponds to 1 kHz, the phase 1008-2 of the second evoked response signal remains at a relatively high level. However, the phase 1008-2 suddenly changes to a relatively low level at insertion time T2 as electrode lead 600 passes the characteristic frequency location that corresponds to 1 kHz.

The decreasing of the second evoked response amplitude 1006-2 and the changing of phase 1008-2 from the high level to the low level in Fig. 10 occur at substantially the same insertion time T2, and both occur as electrode 604-1 passes the characteristic frequency location that corresponds to 1 kHz. Hence, diagnostic system 300 may determine that electrode 604-1 passes the characteristic frequency location that corresponds to 1 kHz by detecting, within an additional predetermined time period, that both an amplitude 1006-2 of the second evoked response signal recorded by electrode 604-1 decreases by at least an amplitude threshold amount and a phase 1008-2 of the second evoked response signal recorded by electrode 604-1 changes by at least a phase threshold amount. The additional predetermined time period, the amplitude threshold amount, and/or the phase threshold amount may each be set by diagnostic system 300 to be any suitable value, such as described herein.

As is further shown in Fig. 10, after electrode lead 600 passes the characteristic frequency location that corresponds to 1 kHz, electrode lead 600 advances towards the characteristic frequency location that corresponds to 500 Hz. As electrode lead 600 advances toward the characteristic frequency location that corresponds to 500 Hz, the amplitude 1006-3 of the third evoked response signal, which is generated in response to acoustic stimulation having a stimulus frequencies of 500 Hz, increases and peaks at insertion time T3 when electrode lead 600 is positioned at position P3. As electrode lead 600 passes the characteristic frequency location that corresponds to 500 Hz, the third evoked response amplitude 1006-3 decreases until it settles at a steady state value. As shown in graph 1004, as electrode lead 600 advances towards the characteristic frequency location that corresponds to 500 Hz, the phase 1008-3 of the third evoked response signal remains at a relatively high level. However, the phase 1008-3 suddenly changes to a relatively low level at insertion time T3 as electrode lead 600 passes the characteristic frequency location that corresponds to 500 Hz.

The decreasing of the third evoked response amplitude 1006-3 and the changing of phase 1008-3 from the high level to the low level in Fig. 10 occur at substantially the same insertion time T3, and both occur as electrode 604-1 passes the characteristic frequency location that corresponds to 500 Hz. Hence, diagnostic system 300 may determine that electrode 604-1 passes the characteristic frequency location that corresponds to 500 Hz by detecting, within an additional predetermined time period, that both an amplitude 1006-3 of the third evoked response signal recorded by electrode 604-1 decreases by at least an amplitude threshold amount and a phase 1008-3 of the third evoked response signal recorded by electrode 604-1 changes by at least a phase threshold amount. The additional predetermined time period, the amplitude threshold amount, and/or the phase threshold amount may each be set by diagnostic system 300 to be any suitable value, such as described herein.

In certain examples, diagnostic system 300 may perform similar operations such as those described herein to determine when electrode lead passes other characteristic frequency locations that correspond to other frequencies (e.g., 4 kHz, 250 Hz, etc.).

In certain examples, diagnostic system 300 may determine that electrode lead 600 passes a characteristic frequency based on at least one of an amplitude of an additional evoked response signal included in the plurality of evoked response signals not decreasing by at least the amplitude threshold amount and a phase of the additional evoked response signal not changing by at least the phase threshold amount. For example, diagnostic system 300 may determine that electrode lead 600 passes the characteristic frequency location that corresponds to 1 kHz based on amplitude 1006-3 of the third evoked response signal not decreasing by an amplitude threshold amount and/or phase 1008-3 not changing by at least a phase threshold amount at insertion time T2 in addition to amplitude 1006-2 and phase 1008-3 of the second evoked response signal changing by an amplitude threshold amount and a phase threshold amount at insertion time T2.

In Fig. 10, various aspects of electrode lead 600 and the illustrated anatomical features of the recipient are simplified for clarity of illustration. For instance, while cochlea 602 has been "unrolled" in Fig. 10, it will be understood that cochlea 602 has a curved, spiral-shaped structure and that electrode lead 600 curves to follow the spiral-shaped structure. Similarly, the anatomy of cochlea 602 omit many details and are not drawn to scale.

Fig. 10 does, however, illustrate at least one additional structure that may be associated with an insertion state that may be determined by diagnostic system 300. In particular, Fig. 10 also shows a basilar membrane 1010 that extends along a length of cochlea 602. As electrode lead 600 is inserted along cochlea 602, electrode lead 600 may contact a structure of cochlea 602 such as basilar membrane 1010. In such examples, diagnostic system 300 may determine that electrode lead 600 is in contact with the structure of cochlea 602 when amplitudes of at least two of the evoked response signals included in the plurality of evoked response signals have decreased by at least an amplitude threshold amount and phases of the at least two of the evoked response signals have changed by at least a phase threshold amount.

In certain alternative examples, diagnostic system 300 may determine that electrode lead 600 is in contact with the structure of cochlea 602 when amplitudes of each of the evoked response signals included in the plurality of evoked response signals have decreased by at least an amplitude threshold amount and phases of each of the evoked response signals have changed by at least a phase threshold amount.

Diagnostic system 300 may determine any suitable number and/or type of insertion states as may serve a particular implementation. In certain examples, an insertion state may correspond to the electrode lead passing a particular characteristic frequency location within the cochlea. In such examples, diagnostic system 300 may determine that the electrode lead passes the particular characteristic frequency location when, within a predetermined amount of time, both an amplitude of a particular evoked response signal included in the plurality of evoked response signals decreases by at least an amplitude threshold amount and a phase of the particular evoked response signal changes by at least a phase threshold amount. The particular characteristic frequency location may correspond to a particular stimulus frequency that corresponds to the particular evoked response signal and that is included in the plurality of stimulus frequencies. Accordingly, diagnostic system 300 may determine an insertion state as passing a certain characteristic frequency location based on which evoked response signal has both a decrease in amplitude by at least an amplitude threshold amount and a phase change by at least a phase threshold amount.

To illustrate, Fig. 10 shows an exemplary lead insertion procedure in which electrode lead 600 is advanced into cochlea 602. Reference numbers P1 through P3 indicate positions of electrode lead 600. For example, at position P1, electrode lead 600 is at a first position in which electrode 604-1 is at the characteristic frequency location that corresponds to 2 kHz. At position P2, electrode lead 600 is at a second position in which electrode 604-1 is at the characteristic frequency location that corresponds to 1 kHz. At position P3, electrode lead 600 is at a third position in which electrode 604-1 is at the characteristic frequency location that corresponds to 500 Hz.

Fig. 10 also shows a graph 1002 of amplitudes 1006 (e.g., amplitudes 1006-1 through 1006-3) of evoked response signals recorded by electrode 604-1 at different insertion times T (e.g., T1 through T3) during the lead insertion procedure. In addition, Fig. 10 shows a graph 1004 of phases 1008 (e.g., phases 1008-1 through 1008-3) of the evoked response signals recorded by electrode 604-1 at different insertion times T during the lead insertion procedure. In this example, first, second, and third evoked response signals are generated in response to acoustic stimulation having stimulus frequencies of 2 kHz, 1 kHz, and 500 Hz, respectively. Hence, as shown in graph 1002, as electrode lead 600 advances towards the characteristic frequency location that corresponds to 2 kHz, the amplitude 1006-1 of a first evoked response signal, which is generated in response to acoustic stimulation having a stimulus frequencies of 2 kHz, increases and peaks at insertion time T1 when electrode lead 600 is positioned at position P1. As electrode lead 600 passes the characteristic frequency location that corresponds to 2 kHz, the first evoked response amplitude 1006-1 decreases until it settles at a steady state value. As shown in graph 1004, as electrode lead 600 advances towards the characteristic frequency location that corresponds to 2 kHz, the phase 1008-1 of the first evoked response signal remains at a relatively high level. However, the phase 1008-1 suddenly changes to a relatively low level at insertion time T1 as electrode lead 600 passes the characteristic frequency location that corresponds to 2 kHz.

As shown in Fig. 10, the decreasing of the first evoked response amplitude 1006-1 and the changing of the phase 1008-1 from the high level to the low level occur at substantially the same insertion time T1, and both occur as electrode 604-1 passes the characteristic frequency location that corresponds to 2 kHz. Hence, diagnostic system 300 may determine that electrode 604-1 passes the characteristic frequency location that corresponds to 2 kHz by detecting, within a predetermined time period, that both an amplitude 1006-1 of the first evoked response signal recorded by electrode 604-1 decreases by at least an amplitude threshold amount and a phase 1008-1 of the first evoked response signal recorded by electrode 604-1 changes by at least a phase threshold amount. The predetermined time period, the amplitude threshold amount, and/or the phase threshold amount may each be set by diagnostic system 300 to be any suitable value. For example, the predetermined time period may be set to be a relatively short time period (e.g., less than a few milliseconds) to ensure that the change in amplitude and in phase correspond to one another. In some examples, diagnostic system 300 may set the predetermined time period, the amplitude threshold amount, and/or the phase threshold in response to user input (e.g., by way of a graphical user interface). Additionally or alternatively, diagnostic system 300 may set the predetermined time period, the amplitude threshold amount, and/or the phase threshold automatically based on one or more factors, such as hearing loss, the stimulus frequency, recipient characteristics (e.g., age, gender, etc.), etc.

As is further shown in Fig. 10, after electrode lead 600 passes the characteristic frequency location that corresponds to 2 kHz, electrode lead 600 advances towards the characteristic frequency location that corresponds to 1 kHz. As electrode lead 600 advances toward the characteristic frequency location that corresponds to 1 kHz, the amplitude 1006-2 of the second evoked response signal, which is generated in response to acoustic stimulation having a stimulus frequencies of 1 kHz, increases and peaks at insertion time T2 when electrode lead 600 is positioned at position P2. As electrode lead 600 passes the characteristic frequency location that corresponds to 1 kHz, the second evoked response amplitude 1006-2 decreases until it settles at a steady state value. As shown in graph 1004, as electrode lead 600 advances towards the characteristic frequency location that corresponds to 1 kHz, the phase 1008-2 of the second evoked response signal remains at a relatively high level. However, the phase 1008-2 suddenly changes to a relatively low level at insertion time T2 as electrode lead 600 passes the characteristic frequency location that corresponds to 1 kHz.

The decreasing of the second evoked response amplitude 1006-2 and the changing of phase 1008-2 from the high level to the low level in Fig. 10 occur at substantially the same insertion time T2, and both occur as electrode 604-1 passes the characteristic frequency location that corresponds to 1 kHz. Hence, diagnostic system 300 may determine that electrode 604-1 passes the characteristic frequency location that corresponds to 1 kHz by detecting, within an additional predetermined time period, that both an amplitude 1006-2 of the second evoked response signal recorded by electrode 604-1 decreases by at least an amplitude threshold amount and a phase 1008-2 of the second evoked response signal recorded by electrode 604-1 changes by at least a phase threshold amount. The additional predetermined time period, the amplitude threshold amount, and/or the phase threshold amount may each be set by diagnostic system 300 to be any suitable value, such as described herein.

As is further shown in Fig. 10, after electrode lead 600 passes the characteristic frequency location that corresponds to 1 kHz, electrode lead 600 advances towards the characteristic frequency location that corresponds to 500 Hz. As electrode lead 600 advances toward the characteristic frequency location that corresponds to 500 Hz, the amplitude 1006-3 of the third evoked response signal, which is generated in response to acoustic stimulation having a stimulus frequencies of 500 Hz, increases and peaks at insertion time T3 when electrode lead 600 is positioned at position P3. As electrode lead 600 passes the characteristic frequency location that corresponds to 500 Hz, the third evoked response amplitude 1006-3 decreases until it settles at a steady state value. As shown in graph 1004, as electrode lead 600 advances towards the characteristic frequency location that corresponds to 500 Hz, the phase 1008-3 of the third evoked response signal remains at a relatively high level. However, the phase 1008-3 suddenly changes to a relatively low level at insertion time T3 as electrode lead 600 passes the characteristic frequency location that corresponds to 500 Hz.

The decreasing of the third evoked response amplitude 1006-3 and the changing of phase 1008-3 from the high level to the low level in Fig. 10 occur at substantially the same insertion time T3, and both occur as electrode 604-1 passes the characteristic frequency location that corresponds to 500 Hz. Hence, diagnostic system 300 may determine that electrode 604-1 passes the characteristic frequency location that corresponds to 500 Hz by detecting, within an additional predetermined time period, that both an amplitude 1006-3 of the third evoked response signal recorded by electrode 604-1 decreases by at least an amplitude threshold amount and a phase 1008-3 of the third evoked response signal recorded by electrode 604-1 changes by at least a phase threshold amount. The additional predetermined time period, the amplitude threshold amount, and/or the phase threshold amount may each be set by diagnostic system 300 to be any suitable value, such as described herein.

In certain examples, diagnostic system 300 may perform similar operations such as those described herein to determine when electrode lead passes other characteristic frequency locations that correspond to other frequencies (e.g., 4 kHz, 250 Hz, etc.).

In certain examples, diagnostic system 300 may determine that electrode lead 600 passes a characteristic frequency based on at least one of an amplitude of an additional evoked response signal included in the plurality of evoked response signals not decreasing by at least the amplitude threshold amount and a phase of the additional evoked response signal not changing by at least the phase threshold amount. For example, diagnostic system 300 may determine that electrode lead 600 passes the characteristic frequency location that corresponds to 1 kHz based on amplitude 1006-3 of the third evoked response signal not decreasing by an amplitude threshold amount and/or phase 1008-3 not changing by at least a phase threshold amount at insertion time T2 in addition to amplitude 1006-2 and phase 1008-3 of the second evoked response signal changing by an amplitude threshold amount and a phase threshold amount at insertion time T2.

In Fig. 10, various aspects of electrode lead 600 and the illustrated anatomical features of the recipient are simplified for clarity of illustration. For instance, while cochlea 602 has been "unrolled" in Fig. 10, it will be understood that cochlea 602 has a curved, spiral-shaped structure and that electrode lead 600 curves to follow the spiral-shaped structure. Similarly, the anatomy of cochlea 602 omit many details and are not drawn to scale.

Fig. 10 does, however, illustrate at least one additional structure that may be associated with an insertion state that may be determined by diagnostic system 300. In particular, Fig. 10 also shows a basilar membrane 1010 that extends along a length of cochlea 602. As electrode lead 600 is inserted along cochlea 602, electrode lead 600 may contact a structure of cochlea 602 such as basilar membrane 1010. In such examples, diagnostic system 300 may determine that electrode lead 600 is in contact with the structure of cochlea 602 when amplitudes of at least two of the evoked response signals included in the plurality of evoked response signals have decreased by at least an amplitude threshold amount and phases of the at least two of the evoked response signals have changed by at least a phase threshold amount.

In certain alternative examples, diagnostic system 300 may determine that electrode lead 600 is in contact with the structure of cochlea 602 when amplitudes of each of the evoked response signals included in the plurality of evoked response signals have decreased by at least an amplitude threshold amount and phases of each of the evoked response signals have changed by at least a phase threshold amount.

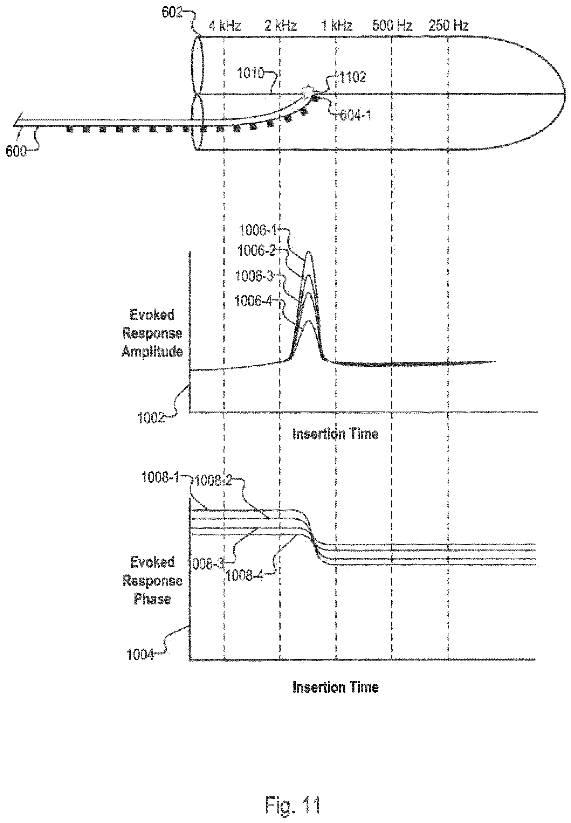

To illustrate, Fig. 11 shows an exemplary electrode lead insertion procedure in which electrode lead 600 is advanced into cochlea 602. Fig. 11 also shows graph 1002 of amplitudes 1006 (e.g., amplitudes 1006-1 through 1006-4) of evoked response signals recorded by electrode 604-1 during the lead insertion procedure. In addition, Fig. 11 shows graph 1004 of phases 1008 (e.g., phases 1008-1 through 1008-4) of the evoked response signals recorded by electrode 604-1 during the lead insertion procedure. In this example, first, second, third, and fourth evoked response signals are generated in response to acoustic stimulation having stimulus frequencies of 2 kHz, 1 kHz, 500 Hz, and 250 Hz, respectively.

As shown in Fig. 11, electrode lead 600 has come into contact with basilar membrane 1010 at a position 1102 along the length of basilar membrane 1010. Hence, as shown in graph 1002, amplitudes 1006 of each of the first, second, third, and fourth evoked response signals increase and peak as a result of electrode lead 600 contacting basilar membrane 1010 at position 1102. In addition, as shown in graph 1004, phase 1008 of each of the first, second, third, and fourth evoked response signals change from a relatively high level to a relatively low level as a result of electrode lead 600 contacting basilar membrane 1010 at position 1102.

As shown in Fig. 11, the decreasing of the first, second, third, and fourth evoked response amplitudes 1006 and the changing of each of phases 1008 from the high level to the low level occur at substantially the same time (e.g., within a predetermined time period), and each occur as electrode lead 600 contacts basilar membrane 1010 and changes mechanical stiffness of basilar membrane 1010. Hence, diagnostic system 300 may determine that electrode lead 600 contacts a structure such as basilar membrane 1010 by determining, within a predetermined time period, that the amplitudes of each of the evoked response signals included in the plurality of evoked response signals have decreased by at least the amplitude threshold amount and the phases of each of the evoked response signals have changed by at least the phase threshold amount. The predetermined time period, the amplitude threshold amount, and/or the phase threshold amount may each be set by diagnostic system 300 to be any suitable value, such as described herein.

In certain examples, diagnostic system 300 may be configured to determine a location and/or an amount of contact with respect to the structure of cochlea 602 based on amplitudes and phases of each of the evoked response signals. The location of the contact may be determined in any suitable manner. In addition, the amount of contact may be determined in any suitable manner. For example, amplitudes 1006 of each of the first, second, third, and fourth evoked response signals shown in Fig. 11 may be indicative of a first amount of contact with respect to basilar membrane 1010 at position 1102. Relatively larger amplitudes 1006 of each of the first, second, third, and fourth evoked response signals may be indicative of a second amount of contact with respect to basilar membrane 1010 at position 1102 that is relatively greater than the first amount of contact. Additionally or alternatively, an amount of phase change may be indicative of an amount of contact with respect to basilar membrane 1010 at position 1102. For example, the amount of phase change shown in Fig. 11 may be indicative of electrode lead 600 contacting basilar membrane 1010 at a first amount of contact. The amount of phase change may increase with greater contact and/or in response to electrode lead 600 translocating basilar membrane 1010.

In certain examples, an insertion state of an electrode lead may be associated with an electrode lead passing a cluster of a particular type of cells (e.g., hair cells, neuron cells, etc.) within the cochlea. In such examples, diagnostic system 300 may determine that an electrode lead passes a cluster of a particular type of cells such as hair cells when amplitudes of one or more evoked response signals included in the plurality of evoked response signals have decreased by at least an amplitude threshold amount and phases of the one or more evoked response signals have not changed by at least a phase threshold amount. For example, diagnostic system 300 may determine that the electrode lead passes a cluster of hair cells when the amplitudes of the second, third, and fourth evoked response signals decrease by at least an amplitude threshold amount and the phases of the second, third, and fourth evoked response signals do not change by at least a phase threshold amount.

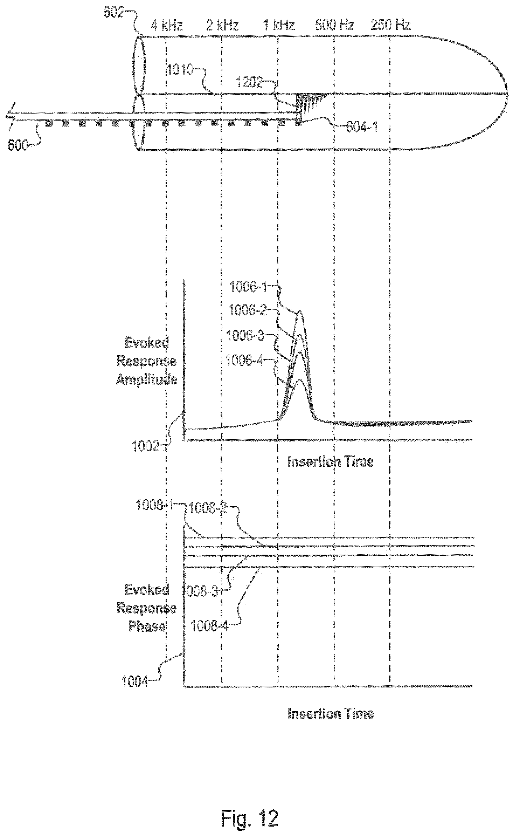

In certain alternative examples, diagnostic system 300 may determine that an electrode lead passes a cluster of a particular type of cells such as hair cells when amplitudes of each of the evoked response signals included in the plurality of evoked response signals have decreased by at least an amplitude threshold amount and phases of each of the evoked response signals have not changed by at least a phase threshold amount. To illustrate an example, Fig. 12 shows an exemplary electrode lead insertion procedure in which electrode lead 600 is advanced into cochlea 602. Fig. 12 also shows graph 1002 of amplitudes 1006 (e.g., amplitudes 1006-1 through 1006-4) of evoked response signals recorded by electrode 604-1 during the lead insertion procedure. In addition, Fig. 12 shows graph 1004 of phases 1008 (e.g., phases 1008-1 through 1008-4) of the evoked response signals recorded by electrode 604-1 during the lead insertion procedure. In this example, first, second, third, and fourth evoked response signals are generated in response to acoustic stimulation having stimulus frequencies of 2 kHz, 1 kHz, 500 Hz, and 250 Hz, respectively.

As shown in Fig. 12, electrode lead 600 passes a cluster of hair cells 1202 along the length of cochlea 602. As a result of passing cluster of hair cells 1202, an amplitude 1006 of each of first, second, third, and fourth evoked response signals has peaked and dropped at an insertion time associated with passing cluster of hair cells 1202. However, as shown in graph 1004, phases 1008 of each of the first, second, third, and fourth evoked response signals have not changed by at least a phase threshold amount as a result of passing cluster of hair cells 1202. Hence, diagnostic system 300 may determine that electrode lead 600 passes cluster of hair cells 1202 when, within a predetermined time period, amplitudes of one or more evoked response signals included in the plurality of evoked response signals have decreased by at least an amplitude threshold amount and that phases of the one or more evoked response signals have not changed by at least a phase threshold amount.

In certain examples, an insertion state of an electrode lead may be associated with a possible occurrence of trauma (e.g., translocation from the scala tympani to the scala vestibuli (i.e., by penetrating through the basilar membrane)) to a structure of a cochlea of a recipient. Such trauma may be caused by the electrode lead penetrating the basilar membrane of the cochlea, inadvertently being placed within a wrong duct of the cochlea, and/or in any other suitable manner. In such examples, diagnostic system 300 may determine that the electrode lead has caused trauma to the cochlea based on a determination that amplitudes of evoked response signals included in a plurality of evoked response signals have decreased by at least an amplitude threshold amount and phases of the evoked response signals have changed by at least a phase threshold amount that is relatively larger than an additional phase threshold amount indicative of the electrode lead merely contacting a structure of the cochlea. In this regard, diagnostic system 300 may use different phase threshold amounts to determine different insertion states of electrode lead 600 in certain implementations.

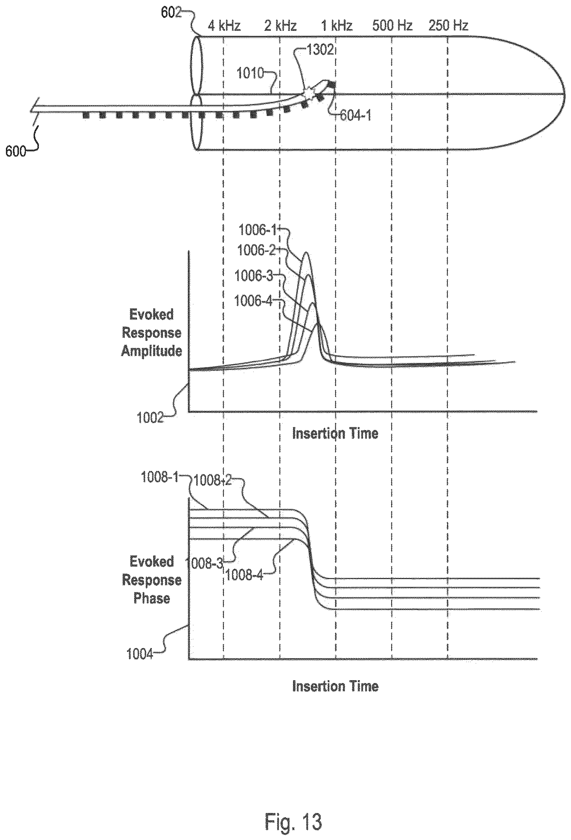

To illustrate, Fig. 13 shows an exemplary electrode lead insertion procedure in which electrode lead 600 is advanced into cochlea 602. Fig. 13 also shows graph 1002 of amplitudes 1006 (e.g., amplitudes 1006-1 through 1006-4) of evoked response signals recorded by electrode 604-1 during the lead insertion procedure. In addition, Fig. 13 shows graph 1004 of phases 1008 (e.g., phases 1008-1 through 1008-4) of the evoked response signals recorded by electrode 604-1 during the lead insertion procedure. In this example, first, second, third, and fourth evoked response signals are generated in response to acoustic stimulation having stimulus frequencies of 2 kHz, 1 kHz, 500 Hz, and 250 Hz, respectively.

As shown in Fig. 13, electrode lead 600 has come into contact with and has punctured basilar membrane 1010 at a position 1302 along the length of basilar membrane 1010. Hence, as shown in graph 1002, amplitudes 1006 of each of the first, second, third, and fourth evoked response signals increase and peak decrease by at least an amplitude threshold amount with respect to each other as a result of electrode lead 600 puncturing basilar membrane 1010 at position 1302. As shown in graph 1004, phase 1008 of each of the first, second, third, and fourth evoked response signals change from a relatively high level to a relatively low level as a result of electrode lead 600 puncturing basilar membrane 1010. The amount of phase change shown in Fig. 13 is relatively larger than the amount of phase change shown in Fig. 11. This is because there is a relatively higher phase change threshold associated with electrode lead 600 causing trauma to cochlea 602 as compared to electrode lead 600 merely contacting basilar membrane 1010, as shown in Fig. 11.

As shown in Fig. 13, the decreasing of the first, second, third, and fourth evoked response amplitudes 1006 by at least the amplitude threshold amount and the changing of each of phases 1008 from the high level to the low level occur at substantially the same time, and each occur as electrode 604-1 punctures basilar membrane 1010. Hence, diagnostic system 300 may determine that electrode 604-1 has caused trauma to cochlea 602 based on diagnostic system 300 determining, within a predetermined time period, that the amplitudes of the evoked response signals included in the plurality of evoked response signals have decreased by at least the amplitude threshold amount and the phases of the evoked response signals changing have changed by at least a phase threshold amount that is relatively larger than an additional phase threshold amount indicative of the electrode lead contacting a structure of the cochlea. The phase threshold amount associated with causing trauma to cochlea 602 may be set by diagnostic system 300 to be any suitable value, such as described herein.

In certain examples, evoked response amplitudes (e.g., evoked response amplitudes 1006) decreasing by different amounts with respect to each other may additionally or alternatively be indicative of an electrode lead causing trauma to the cochlea. Any suitable amount difference in the decrease of the amplitudes of the evoked response signals may be indicative of trauma to the cochlea.

An illustrative EFI measurement procedure is described more fully in U.S. Patent No. US11376431, the contents of which are incorporated herein by reference. As used herein, an EFI measurement procedure may include applying stimulation to a particular electrode included on an electrode lead and obtaining EFI data. EFI data refers to data representative of intracochlear potentials (e.g., voltage levels) recorded by other electrodes included in the electrode lead that occur in response to stimulation of the electrode. The EFI data may be obtained in any suitable manner.

For example, diagnostic system 300 may obtain EFI data for a monopolar stimulation configuration associated with an electrode included in an electrode lead by directing a cochlear implant included in the cochlear implant system to apply a stimulation pulse to the electrode using a monopolar stimulation configuration. Diagnostic system 300 may then record an intracochlear potential that occurs in response to the stimulation pulse at each of a remaining number of electrodes in the electrode lead to obtain an EFI data set corresponding to the monopolar stimulation configuration.

EFI data may be used in any suitable manner. For example, anomalies in EFI data may be indicative of tip fold over and/or any other insertion state of the electrode lead.

As used herein, an "excitation spread measurement" may refer to any measurement configured to determine the extent to which stimulation (e.g., an electrical pulse) applied by one electrode at one location may spread or travel (e.g., through fluid and/or tissue at and surrounding the location) so as to be detectable (e.g., as a voltage) by another electrode at another location. As such, an excitation spread measurement as performed by the systems and methods described herein may be similar to a conventional impedance measurement in which stimulation is applied by an electrode and then detected by the same electrode (e.g., with reference to a ground electrode, with reference to another separate stimulating electrode, etc.). However, in contrast with conventional impedance measurements, excitation spread measurements as performed by the systems and methods described herein may apply stimulation with a different and distinct electrode from the electrode used to record (e.g., detect) the stimulation (e.g., a voltage resulting from the application of the stimulation) as the stimulation spreads. As such, in some examples, an excitation spread measurement may also be referred to as a "cross impedance" measurement or the like. Excitation spread measurements are described more fully in U.S. Patent Publication No. US20220305264A1, the contents of which are incorporated herein by reference.

As one example of how an excitation spread measurement may be performed, diagnostic system 300 may direct a first electrode (e.g., electrode 604-1) to generate an electrical pulse and, in response to the generation of the electrical pulse, may detect a voltage between a second electrode (e.g., another one of electrodes 604 or a ground electrode) and a reference (e.g., a ground electrode, a case ground of a cochlear implant, etc.) where both the second electrode and the reference are distinct from the first electrode. Based on the excitation spread measurement, diagnostic system 300 may determine whether at least one of the first electrode or the second electrode is located within cochlea 602.

In some examples, a non-transitory computer-readable medium storing computer-readable instructions may be provided in accordance with the principles described herein. The instructions, when executed by a processor of a computing device, may direct the processor and/or computing device to perform one or more operations, including one or more of the operations described herein. Such instructions may be stored and/or transmitted using any of a variety of known computer-readable media.

A non-transitory computer-readable medium as referred to herein may include any non-transitory storage medium that participates in providing data (e.g., instructions) that may be read and/or executed by a computing device (e.g., by a processor of a computing device). For example, a non-transitory computer-readable medium may include, but is not limited to, any combination of non-volatile storage media and/or volatile storage media. Exemplary non-volatile storage media include, but are not limited to, read-only memory, flash memory, a solid-state drive, a magnetic storage device (e.g. a hard disk, a floppy disk, magnetic tape, etc.), ferroelectric random-access memory ("RAM"), and an optical disc (e.g., a compact disc, a digital video disc, a Blu-ray disc, etc.). Exemplary volatile storage media include, but are not limited to, RAM (e.g., dynamic RAM).

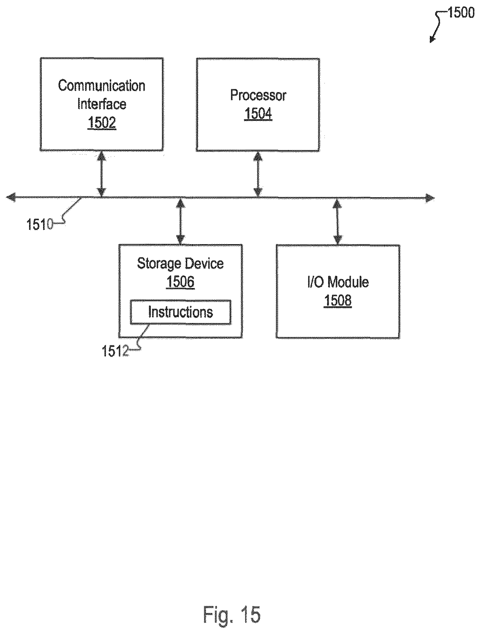

Fig. 15 illustrates an exemplary computing device 1500 that may be specifically configured to perform one or more of the processes described herein. As shown in Fig. 15, computing device 1500 may include a communication interface 1502, a processor 1504, a storage device 1506, and an input/output ("I/O") module 1508 communicatively connected one to another via a communication infrastructure 1510. While an exemplary computing device 1500 is shown in Fig. 15, the components illustrated in Fig. 15 are not intended to be limiting. Additional or alternative components may be used in other embodiments. Components of computing device 1500 shown in Fig. 15 will now be described in additional detail.

Communication interface 1502 may be configured to communicate with one or more computing devices. Examples of communication interface 1502 include, without limitation, a wired network interface (such as a network interface card), a wireless network interface (such as a wireless network interface card), a modem, an audio/video connection, and any other suitable interface.

Processor 1504 generally represents any type or form of processing unit capable of processing data and/or interpreting, executing, and/or directing execution of one or more of the instructions, processes, and/or operations described herein. Processor 1504 may perform operations by executing computer-executable instructions 1512 (e.g., an application, software, code, and/or other executable data instance) stored in storage device 1506.

Storage device 1506 may include one or more data storage media, devices, or configurations and may employ any type, form, and combination of data storage media and/or device. For example, storage device 1506 may include, but is not limited to, any combination of the non-volatile media and/or volatile media described herein. Electronic data, including data described herein, may be temporarily and/or permanently stored in storage device 1506. For example, data representative of computer-executable instructions 1512 configured to direct processor 1504 to perform any of the operations described herein may be stored within storage device 1506. In some examples, data may be arranged in one or more databases residing within storage device 1506.

I/O module 1508 may include one or more I/O modules configured to receive user input and provide user output. I/O module 1508 may include any hardware, firmware, software, or combination thereof supportive of input and output capabilities. For example, I/O module 1508 may include hardware and/or software for capturing user input, including, but not limited to, a keyboard or keypad, a touchscreen component (e.g., touchscreen display), a receiver (e.g., an RF or infrared receiver), motion sensors, and/or one or more input buttons.

I/O module 1508 may include one or more devices for presenting output to a user, including, but not limited to, a graphics engine, a display (e.g., a display screen), one or more output drivers (e.g., display drivers), one or more audio speakers, and one or more audio drivers. In certain embodiments, I/O module 1508 is configured to provide graphical data to a display for presentation to a user. The graphical data may be representative of one or more graphical user interfaces and/or any other graphical content as may serve a particular implementation.

In some examples, any of the systems, computing devices, and/or other components described herein may be implemented by computing device 1500. For example, storage facility 302 may be implemented by storage device 1506, and processing facility 304 may be implemented by processor 1504.

In the preceding description, various exemplary embodiments have been described with reference to the accompanying drawings. It will, however, be evident that various modifications and changes may be made thereto, and additional embodiments may be implemented, without departing from the scope of the invention as set forth in the claims that follow. For example, certain features of one embodiment described herein may be combined with or substituted for features of another embodiment described herein. The description and drawings are accordingly to be regarded in an illustrative rather than a restrictive sense.

## Claims

1. A system comprising:
a memory storing instructions; and
one or more processors communicatively coupled to the memory and configured to execute the instructions to perform a process comprising:
performing, during a lead insertion procedure during which an electrode lead is inserted into a cochlea of a recipient of a cochlear implant, a first evoked response-based measurement test that generates first result data;
determining that the first result data satisfies a first condition;
performing, based on the first result data satisfying the first condition and during the lead insertion procedure, a second evoked response-based measurement test that generates second result data, the second evoked response-based measurement test being of a different type than the first evoked response-based measurement test; and
performing, based on the second result data, an operation associated with the lead insertion procedure.

2. The system of claim 1, wherein the performing the second evoked response-based measurement test is automatically initiated without user input being provided to initiate the second evoked response-based measurement test.

3. The system of claim 1, wherein the performing the operation is further based on the first result data.

4. The system of claim 1, wherein the performing the operation comprises:
determining that the second result data satisfies a second condition; and
performing, based on the second result data satisfying the second condition and during the lead insertion procedure, a third evoked response-based measurement test that generates third result data.

5. The system of claim 4, wherein the performing the operation is further based on the third result data.

6. The system of claim 1, wherein the performing the operation comprises providing, during the lead insertion procedure, a notification associated with the lead insertion procedure.

7. The system of claim 1, wherein the performing the operation comprises providing, during the lead insertion procedure, feedback to a robotic lead insertion system, the feedback configured to adjust one or more operating parameters of the robotic lead insertion system.

8. The system of claim 1, wherein the performing the operation comprises adjusting one or more parameters configured to control the lead insertion procedure.

9. The system of claim 1, further comprising:
selecting, by the system based on the first result data, a particular evoked response-based measurement test from a plurality of available evoked response-based measurement tests; and
designating, by the system, the particular evoked response-based measurement test as the second evoked response-based measurement test.

10. The system of claim 9, further comprising:
providing, by the system, the first result data as an input to a machine learning model;
wherein the selecting the particular evoked response-based measurement test is based on an output of the machine learning model.

11. The system of claim 10, wherein the machine learning model is trained using historical test data associated with one or more lead insertion procedures performed prior to the lead insertion procedure.

12. The system of claim 1, wherein:
the first evoked response-based measurement test comprises a single tone-based insertion depth monitoring procedure that measures an evoked response that occurs in response to application of acoustic stimulation having a single frequency; and
the second first evoked response-based measurement test comprises at least one of a multi tone-based insertion depth monitoring procedure that measures multiple evoked responses that occur in response to application of an additional acoustic stimulation having multiple frequencies or an electric field imaging measurement procedure that measures one or more evoked responses that occur in response to electrical stimulation being applied to one or more electrodes of the electrode lead.

13. A method comprising:
performing, by a diagnostic system during a lead insertion procedure during which an electrode lead is inserted into a cochlea of a recipient of a cochlear implant, a first evoked response-based measurement test that generates first result data;
determining, by the diagnostic system, that the first result data satisfies a first condition;
performing, by the diagnostic system based on the first result data satisfying the first condition and during the lead insertion procedure, a second evoked response-based measurement test that generates second result data, the second evoked response-based measurement test being of a different type than the first evoked response-based measurement test; and
performing, by the diagnostic system based on the second result data, an operation associated with the lead insertion procedure.

14. The method of claim 13, wherein the performing the second evoked response-based measurement test is automatically initiated by the diagnostic system without user input being provided to initiate the second evoked response-based measurement test.

15. A non-transitory computer-readable medium storing instructions that, when executed, direct a processor of a computing device to perform a process comprising:
performing, during a lead insertion procedure during which an electrode lead is inserted into a cochlea of a recipient of a cochlear implant, a first evoked response-based measurement test that generates first result data;
determining that the first result data satisfies a first condition;
performing, based on the first result data satisfying the first condition and during the lead insertion procedure, a second evoked response-based measurement test that generates second result data, the second evoked response-based measurement test being of a different type than the first evoked response-based measurement test; and
performing, based on the second result data, an operation associated with the lead insertion procedure.
